(19) Europäisches Patentamt
European Patent Office
Office européen des brevets

(11) Publication number: **0 363 903 A2**

(12) # EUROPEAN PATENT APPLICATION

(21) Application number: **89118808.8**

(22) Date of filing: **10.10.89**

(51) Int. Cl.[5]: **A61K 6/08 , A61K 6/09**

(30) Priority: **14.10.88 US 258304**

(43) Date of publication of application: **18.04.90 Bulletin 90/16**

(84) Designated Contracting States: **AT BE CH DE ES FR GB GR IT LI LU NL SE**

(71) Applicant: **DENTSPLY INTERNATIONAL**
**570 West College Avenue**
**York Pennsylvania 17405(US)**

(72) Inventor: **Blackwell, Gordon B.**
**c/o Detrey Dentsply Reichenaustrasse 150**
**D-7750 Konstanz(DE)**
Inventor: **Huang, Chin-Teh**
**22 Crossley Drive**
**Dover Delaware 19901(US)**

(74) Representative: **Wächtershäuser, Günter, Dr.**
**Tal 29**
**D-8000 München 2(DE)**

(54) **Adhesive containing pit and fissure sealants.**

(57) Dental sealant compositions that include an abrasive and optionally a source of elutable fluoride ions is provided. The compositions demonstrate bond strength to teeth that is comparable with the mechanical bond strength achieved by acid etching, and when used with acid etching assures a good bond to the tooth in anomalous areas where the acid etch fails. In an optional embodiment, elutable fluoride ions may be included in the composition to help prevent carious activity.

EP 0 363 903 A2

# ADHESIVE CONTAINING PIT AND FISSURE SEALANTS

## RELATED APPLICATIONS

This application is a continuation-in-part of U.S. Application Serial No. 939,601, filed December 9, 1986, which is a continuation-in-part of U.S. Serial No. 676,135, filed November 29, 1984.

## BACKGROUND OF THE INVENTION

The invention relates to dental sealant compositions that include an adhesive and optionally a source of elutable fluoride ions. A method for treating a tooth comprising coating a tooth with the resin composition of the invention is also provided.

It is most desirable, when filling a tooth cavity with a filling material, such as a polymerizable dental restorative, to ensure good adhesion between the tooth surrounding the cavity and the set (polymerized) filling material since there is thereby obtained a good seal between the set filling material and the tooth which prevents, or at least markedly inhibits, ingress of mouth fluids and bacteria into the filled cavity and thus prevents further decay or loss of the filling material. In order to achieve good adhesion between the filler material and the tooth enamel, it has been recommended to provide a primer or adhesive bonding layer intermediate the filling material and surfaces of a prepared tooth. As disclosed, for example, in Canadian Patent No. 559,648 to Hagger, the bonding intermediate layer, which acts as an adhesive, should present polar groups toward the inorganic crystal lattice (apatite) of the tooth and be anchored by means of a non-polar group in the filling material. In order to accomplish this goal, it was taught to utilize dimethacrylglycerophosphoric acid as a polymerizable intermediate adhesive bonding layer. In practice, the surfaces in a prepared cavity of a tooth were dampened with the dimethacrylglycerophosphoric acid, usually in an alcohol medium, whereafter a paste of the filling material (monomer, polymer and room temperature effective catalyst such as sulfinic acid) was plugged into the cavity. The catalyst then caused simultaneous setting of the adhesive and filling material at the temperature in the mouth. It was also disclosed to dissolve the catalyst in the polymerizable adhesive and to spread the polymerizable adhesive solution on the surface to be bonded.

The technique disclosed in the Hagger patent was effective for bonding tooth enamel, dentin and ivory with the polymerizable filling materials existing at that time.

Another approach for aiding in the secure placement of fillings, that has been in general use for a number of years now, is the approach of first acid etching the dental preparation and then applying a low viscosity resin to penetrate the interiors caused by etching and thus prime or line the dental preparation. The primer layer is then cured, whereafter a higher viscosity polymerizable filling material is applied to the preparation. The primer usually is a lower viscosity form of the same base polymerizable filling material that will be used to fill the tooth, the difference in viscosity being primarily the result of the addition of less filler to the resin. Because the primer is basically the same system as the filling, compatibility has generally not been a severe problem.

However, as the field of polymerizable dental restorative materials has developed a need for still further improved primers and adhesives has become self-evident, particularly with respect to the need for greater bonding strength and reduction of marginal leakage between exposed dentin and enamel surfaces and the restorative filling materials.

This need lead to the development of two part chemically cured adhesives, such as those described in U.S. Patent No. 4,182,035 to Yamauchi et al. and in European Patent application No. 0084407 to Bunker (corresponds to publication number 0058483). The adhesive described in the Yamauchi et al. patent comprises a first package consisting of a free radical polymerizable monomer and a diacyl peroxide, and a second package comprising an alkali or alkaline earth metal salt of an arylsulfinic acid and a volatile solvent. The adhesive disclosed in the Bunker application comprises a polymerizable phosphorous compound, a tertiary amine, a polymerization catalyst, a diluent, and an effective amount of a metal selected from iron, copper, manganese, cobalt, tin, chromium, nickel and zinc dissolved in a polar solvent. The metal dissolved in the polar solvent is stored separately from the remainder of the composition and is added thereto prior to the application of the adhesive to a tooth surface.

While self-curing multi-part systems of the type described above can provide improved adhesion

between a tooth surface and a polymerizable filling material, the limitations associated with the need for storing two or more components in separate containers until immediately before use can not be minimized. For example, the need for mixing several components prior to use requires not only time, but skill, since it is essential that the proper proportions of polymerizable material, catalyst and accelerator be mixed, without the inclusion of air bubbles, which act as a polymerization inhibitor, so as to ensure the formation of a polymerized adhesive having the desired physical characteristics. Then, too, polymerizable adhesives of the above type normally have limited working times available and must be hardened for a predetermined length of time before a polymerizable filling material can be plugged over the adhesive in the cavity. Following this, one must wait until the filling material and the underlying adhesive have hardened sufficiently to allow the filling material to be shaped and finished with burrs and/or abrasives and the like in order that the rigid filling material does not transmit forces which can dislodge and break or exceed the adhesive bond strength of the newly placed two component adhesive to dentin.

Finally, self-curing two part adhesive systems of the type described above, preferably are used with multi-part self-curing filler materials.

One of the recent advances in polymerizable filling systems has been the one component light curing composite filling material that can be cured in from about 5 to about 40 seconds as contrasted to the 5 to 15 minutes of cure time required for the self-curing two component system and does not require dentist office time for mixing. One component light curing filling materials are premixed in the factory to include all catalysts and aotivators in a single component package that is essentially free of air, needs no subsequent mixing and is sold in a light-tight package in order to exclude activating light. These light curing one component composite filling materials may be applied directly into the prepared restorative cavity and are hardened in a matter of seconds by the application of visible or ultraviolet light. This new one component product has brought about a need for new and improved primers and adhesives which are compatible with strong biologically safe adhesion to the tooth and to the new filling materials. Preferred new primers and adhesives would, in most instances, be convenient one component actinic light cure compositions.

Pit and Fissure sealants are used in an effort to reduce the incidence of occlusal caries by their application to enamel, and principally occlusal surfaces of posterior teeth, the rationale being that the sealants flow into and seal pits and fissures in the teeth, thereby preventing their contact with the oral environment. In general, the enamel of the teeth are acid etched before the sealants are applied since prior art sealants do not adhere to tooth structure. Acid etching of the enamel surface provides a means for mechanical retention of the sealant to the tooth.

In some cases the enamel on a particular tooth may be resistant to acid etching, or if the procedure is not carried out correctly, the sealant will fail to be retained by the enamel. Also, sealants are subject to debonding because of water penetration, temperature fluctuation, mechanical stress, occlusion wear, or a combination of these factors.

If a good cover by the sealant is not obtained, or if debonding occurs, microleakage around the sealant, in time, may cause caries to develop.

It has been proposed by Swartz et al in Dental Reviews, Vo. 55, No. 5, September-October 1976, pp 757-777, to add a source of fluoride ions to sealant compositions to provide some protection against caries if microleakage should occur.

Although the anticariogenic properties of fluoride ions are known, and the addition of fluoride to sealant has shown promising results, the addition of fluoride to sealants only provides a partial solution.

It is an object of the present invention to further improve the effectiveness of dental sealant compositions by including a dental adhesive in the dental formulation.

## SUMMARY OF THE INVENTION

The invention comprises a dental resin composition which comprises at least one adhesive promoting agent. Optionally the composition may include a source of elutable fluoride ions. In a preferred embodiment the composition comprises by weight about 40-60% resin monomers, 0.2-10% adhesion promoting compound, 0.05-2.0% photoinitiator, 0.2-2.0% reducing agents, 0-50% vol inorganic fillers, 1.5-2.5% sodium fluoride and 0.05-0.2% stabilizer.

In the method of the invention, a tooth is treated with a dental resin composition which comprises at least one adhesive promoting agent. In a preferred embodiment, the method comprises acid etching the enamel of a tooth, and coating the tooth with a sealant which also comprises an elutable fluoride.

It has been found that the method and composition of the invention have the advantages that, in the

case where the tooth is resistant to acid etching, the adhesive in the sealant composition provides a good bond to the enamel. In tests of the illustrated embodiment of the sealant of the invention, the sealant provides a bond to enamel, without acid etching, which is twice as strong as the bond to unetched enamel obtained by a sealant which contains no adhesive. Also, the addition of fluoride ions to the composition adds further protection against the incidence of caries.

## DETAILED DESCRIPTION

The above and other objects and advantages are accomplished in accordance with the present invention by providing in one aspect, an adhesive composition comprising a free radical polymerizable monomer or prepolymer having ethylenic unsaturation; a phosphorus derivative which is free from halogen atoms covalently or otherwise bonded directly to a phosphorus atom, preferably a polymerizable phosphorus derivative, having ethylenic unsaturation and being free from halogen atoms bonded to a phosphorus atom; a catalyst, preferably a photosensitive catalyst, especially a diketone; a sulfinic acid (or a salt thereof) or an amine (or amine salt), preferably both a sulfinic acid (or salt thereof) and an amine (or salt thereof); and optionally a solvent for the above ingredients. It should be understood that when a phosphorus derivative having ethylenic unsaturation is used, the phosphorus derivative may function as part or all of the polymerizable monomer or prepolymer. The adhesive composition of this invention is in the form of a shelf-stable one component polymerizable system which obviates the difficulties of the prior art and which, when cured, exhibits exceptionally high adhesive bond strengths between tooth or bone surfaces, particularly dentin, and subsequently applied filling materials.

Thus, while it is known that sulfinates have been used in catalytic quantities in self-curing compositions, especially those containing polymerizable partial esters of phosphoric acid, a peroxide and a polymerizable monomer as one component, and an alkali metal salt of an arylsulfinic acid and an amine and solvent therefor as a second component, it was unexpected to find it possible to combine a polymerizable monomer or prepolymer, a polymerizable phosphate which is free from halogen atoms bonded directly to phosphorus, an amine and a sulfinate in a one component system which is both stable and useful as a dentin adhesive when light cured.

As the free radical-polymerizable monomer or prepolymer to be employed in this invention, use may be made of any monomer, dimer, trimer, or other oligomer of the type that is usable in dental applications. Thus, the polymerizable monomer portion of the present adhesive composition generally comprises one or more monofunctional or polyfunctional ethylenically unsaturated monomers or prepolymers, e.g.,dimers, trimers, and other oligomers, or mixtures or copolymers thereof, based on acrylic or methacrylic or itaconic acid, or derivatives thereof, including their esters which can be polymerized by free radical initiation. These materials include, but are not limited to acrylic and methacrylic acid, itaconic acid and the like, acrylic or methacrylic or itaconic acid esters of monohydric or polyhydric alkanols or polyhydric alcohols containing at least one phenyl group. Examples of such compound include monovinylmethacrylates, e.g., methylmethacry late, ethyl acrylate, propyl methacrylate, hydroxyethylmethyacrylate, hydroxypropylmethacrylate, diethylene glycol acrylate, triethylene glycol acrylate, the monoester of trimellitic acid with hydroxyethyl methacrylate, hydroxypropyl itaconate and the like, esters of aliphatic polyhydric alcohols, such as for example, the di- and polyacrylates, the di- and polymethacrylates, and the di- and polyitaconates of alkylene glycols, alkoxylene glycols, alicyclic glycols and higher polyols, such as ethylene glycol, triethylene glycol, tetraethylene glycol, tetramethylene glycol, trimethylolethane, trimethylolpropane, pentaerythritol, dipentaerythritol, tripentaerythritol, and the like, or mixtures of these with each other or with their partially esterified analogs, and their prepolymers, such compound or mixture optionally having free hydroxyl content. Typical compounds of this type, include but are not limited to, trimethylolpropane triacrylate, trimethylolethane triacrylate, trimethylolpropane trimethacrylate, trimethylolethane trimethacrylate, tetramethylene glycol dimethacrylate, ethylene glycol dimethacrylate, triethylene glycol dimethacrylate, tetraethylene glycol diacrylate, tetraethylene glycol dimethacrylate, pentaerythritol diacrylate, pentaerythritol triacrylate, pentaerythritol tetraacrylate, dipentaerythritol diacrylate, dipentaerythritol triacrylate, dipentaerythritol tetraacrylate, dipentaerythritol pentaacrylate, dipentaerythritol hexacrylate, tripentaerythritol octaacrylate, pentaerythritol dimethacrylate, pentaerythritol trimethacrylate, dipentaerythritol dimethacrylate, glycerin trimethacrylate, ethylene glycol dimethacrylate, butanediol dimethacrylate, trimethylolpropane trimethacrylate, tetramethylolmethane tetramethacrylate, bisphenol-A dimethacrylate, bisphenol-A diglycidyl methacrylate, 2,2′-bis-(4- methacryloxyethoxyphenyl) propane and so on.

Also included among the polymerizable monomers which may be used are the vinyl urethane or

urethane-acrylate prepolymers which are well known in the art. These prepolymers are polymerizable by free radical initiation and may be prepared, for example, by reacting an organic diisocyanate or an isocyanate-terminated urethane prepolymer with an ethylenically unsaturated monomer which is reactive with the diisocyanate or urethane prepolymer. These polymers also may be prepared by reacting a hydroxyl-containing material, such as a polyol or a hydroxyl-terminated urethane prepolymer with an ethylenically unsaturated monomer which is reactive with the polyol or hydroxyl-terminated urethane. The urethane prepolymers, which may be linear or branched, carry isocyanate end groups and generally are prepared by reacting a compound having hydroxyl functionality with a molar excess of diisocyanate.

Any of a wide variety of diisocyanates may be used to prepare the isocyanate-terminated urethane prepolymer including aliphatic, cycloaliphatic, heterocyclic, and aromatic diisocyanates, and combinations of these. Examples include, but are not limited to, 2,4-tolylene diisocyanate, 2,6-tolylene diisocyanate, 1,4-phenylene diisocyanate, 2,2,4-trimethyl hexamethylene diisocyanate, hexamethylene diisocyanate, 1,4-naphthalene diisocyanate, 1,5-naphthalene diisocyanate, 4,4′-diphenylmethane diisocyanate, p,p′- diphenyl diisocyanate, butylene-1,4-diisocyanate, ethylene diisocyanate, trimethylene diisocyanate, tetramethylene-1,4-diisocyanate, butylene-2,3-diisocyanate, cyclohexylene-1,2-diisocyanate, methylene-bis-(4-phenyl-isocyanate), diphenyl-3,3′-dimethyl-4,4′-diisocyanate, xylylene diisocyanate, cyclohexane-1,4-diisocyanate, 1-methoxyphenyl-2,4-diisocyanate and the like, and mixtures thereof.

A wide variety of compounds having hydroxyl functionality may be used to form the isocyanate-terminated urethane prepolymers. For example, diols of the structure

$$HO(\underset{R_1}{CH}-\underset{R_2}{CH}-O)_a Ar(O-\underset{R_1}{CH}-\underset{R_2}{CH})_b OH \quad ,$$

may be used,
where $R_1$ and $R_2$ are hydrogen atoms or alkyl groups, e.g., methyl, and Ar is a divalent aromatic group in which each free valency is on an aromatic carbon atom, and where a and b, independently, may be zero or an integer. Other suitable hydroxyl containing compounds include diols and polyols such as ethylene glycol, propylene glycol, triethylene glycol, tetramethylene glycol, trimethylolpropane, pentaerythritol, dipentaerythritol, and the like, or esters of acrylic acid, methacrylic acid or itaconic acid or the like with aliphatic polyhydric alcohols. Among the more preferred hydroxyl containing compounds are the esters of acrylic or methacrylic acid and a hydroxyalkanol of at least two carbon atoms such as hydroxyethyl acrylate, hydroxyethyl methacrylate, hydroxypropyl acrylate, hydroxypropyl methacrylate, hydroxyisopropyl methacrylate, and the like.

Formation of the isocyanate terminated urethane prepolymers may be assisted by the use of a catalyst known in the art to assist polyurethane formation, for example, tertiary amines and metal salts, e.g., tin salts, titanium salts and the like.

To form the vinyl urethane or urethane-acrylate prepolymer starting materials, an isocyanate-terminated urethane prepolymer or a diisocyanate is reacted with an ethylenically unsaturated compound having hydroxyl functionality. These compounds include for example, esters of acrylic acid, methacrylic acid or itaconic acid with aliphatic polyhydric alcohols, such as hydroxyethyl acrylate, hydroxypropyl methacrylate or the like. The resulting vinyl urethanes are well known in the art and are described for example, in U.S. Patent No. 3,629,187 to Waller, U.S. Patent No. 3,759,809 to Carlick et al, U.S. Patent No. 3,709,866 to Waller and U.S. Patent 4,459,193 to Ratcliffe et al, all of these patents being incorporated herein by reference.

Formation of the vinyl urethane prepolymers may be assisted by the use of the same catalysts noted above, namely, tertiary amines and metal salts.

Of course, the foregoing list of polymerizable ethylenically unsaturated monomers and prepolymers is intended to be exemplary only, and other known polymerizable materials can be used in compositions of this invention.

In practice, a mixture of two or more ethylenically unsaturated materials may be, and commonly is, employed. In the preferred aspects of the invention the polymerizable monomer portion of the composition is liquid at ambient temperature, e.g. about 20-25 °C.

The polymerizable monomer portion of the composition generally should comprise from about 0.5 to about 99.998% by weight of the composition, with amounts ranging from about 1 to about 99.98% being preferred, and amounts ranging from about 1.5 to about 99.8% being more preferred.

The phosphorus derivative which is used as an adhesion promoter in the present composition, may

comprise any of the the well known phosphorus-containing adhesion promoters which are free from any halogen atoms covalently or otherwise bonded directly to a phosphorus atom. The phosphorus derivative may be polymerizable or non-polymerizable, however the preferred phosphorus-containing adhesion promoters comprise polymerizable phosphorus materials having ethylenic unsaturation and include, among others, organic esters of one or more acids of phosphorus (hereinafter referred to as phosphorus acid esters), wherein the organic portion of the ester contains at least one polymerizable ethylenically unsaturated group. The organic portion of the ester may be alkenyl, alkenoxy, cycloalkenyl, aralkenyl, or alkenaryl, and preferably may have from 2 to 40 carbon atoms. The organic portion may be straight chain, branched, or cyclic, can contain skeletal hetero atoms, i.e., atoms other than carbon, and can be unsubstituted or substituted with moieties which do not interfere with the free radical polymerization of the phosphorus acid esters.

Examples of saturated and unsaturated phosphorus acid esters which may be used include, but are not limited to, monomers containing phosphoric acid groups such as hydroxyethyl methacrylate monophosphate, 2,2′-bis(x-methacryloxy-x-hydroxypropoxyphenyl) propane diphosphonate (BIS-GMA diphosphonate), BIS-GMA diphosphate, dibutyl phosphite, di-2-ethylhexyl phosphite, di-2-ethylhexyl phosphate, glyceryl-2-phosphate, glycerylphosphoric acid, methacryloxyethyl phosphate, and glyceryl dimethacrylate phosphate. Other suitable polymerizable phosphorus acid esters are disclosed, for example, in U.S. Patent No. 4,499,251 to Omura et al, U.S. Patent No. 4,222,780 to Shibantani et al, U.S. Patent No. 4,235,633 to Tomioka, U.S. Patent No. 4,259,117 to Yamauchi et al, U.S. Patent No, 4,368,043 to Yamauchi et al, and U.S. Serial No. 464,778 to Prodger, Billington & Blackwell filed February 7, 1983, and assigned to the assignee of this invention. Of the polymerizable phosphorus acid compounds disclosed in the above patents and application, each of which is incorporated herein by reference, the preferred compounds are those polyethylenically unsaturated monophosphates of the formula:

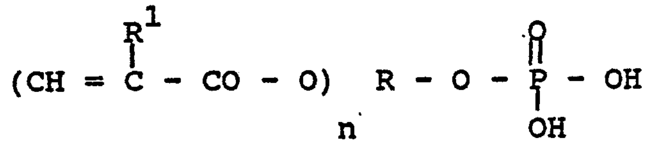

and salts thereof, in which

R is an organic radical having a valency of n + 1; and R may be interrupted by one or more oxygen atoms and may be substituted or unsubstituted, and may comprise an aliphatic radical, or a cycloaliphatic radical, or an aryl radical;

$R^1$ is a hydrogen atom, alkyl $C_1$ - $C_3$, halogen or CN radical, and

n is an integer of at least 1.

Preferably n is an integer of 2 or more, and more preferably from 3 to 6. Examples of the preferred compounds include pentaerythritol triacrylate monophosphate, pentaerythritol trimethacrylate monophosphate, dipentaerythritol pentaacrylate monophosphate, and dipentaerythritol pentamethacrylate monophosphate.

The phosphorus acid compound normally should comprise from about 0.25 to about 99.998% by weight of the adhesive composition, with amounts ranging from about 1 to about 50% being preferred. In a more preferred embodiment the phosphorus acid compound would comprise from about 2 to about 29.8% by weight of the composition.

The catalyst component may comprise any of those free radical initiators normally used in conjunction with polymerizable ethylenically unsaturated materials, although those which will initiate polymeri zation at room temperature are preferred. Thus, the catalyst may comprise, for example, an organic peroxide type initiator such as dibenzoyl peroxide, dilauroyl peroxide, acetyl peroxide, benzoyl peroxide t-butyl peroxybenzoate, cumene hydroperoxide and the like. In a preferred aspect, the catalyst comprises an actinic light sensitive initiator, such as ultraviolet light-sensitive initiators or visible light sensitive initiators. As examples of suitable ultraviolet light-sensitive initiators there may be mentioned the monoketals of an aromatic 1,2-diketone, benzophenones, substituted benzophenones, benzoin methyl ether, isopropoxybenzoin, benzoin phenyl ether or benzoin isobutyl ether. Among the suitable visible light sensitive initiators, -diketones, such as camphoroquinone, are particularly preferred. The preferred initiators are the visible light sensitive initiators.

The catalyst generally is employed in the range of from about 0.001 to about 10% of the composition. In a preferred embodiment the catalyst is used within the range of from 0.01 to about 5%. In a still further

preferred embodiment, from about 0.1 to about 2% by weight of catalyst is employed.

In addition to the foregoing constituents, the adhesive composition also includes an accelerator system comprising (1) an amine or amine salt or (2) a sulfinic acid or salt thereof. In a preferred embodiment both the amine or amine salt and the sulfinic acid or salt thereof are present.

The amine or amine salt may be present in an amount from 0 to about 20% by weight of the composition, whereas the sulfinic acid or salt thereof is present in an amount of from about 0 to about 10%, the combined amount being from about 0.001 to about 20 percent. In a preferred embodiment, the amine or amine salt is used in the range of 0.001 to about 10% by weight and the sulfinic acid or a sulfinic acid salt is used in the range from about 0.01 to about 5%, the combined weight being in the range from about 0.01-15% by weight. In a still more preferred embodiment, the amine or amine salt is used in an amount from about 0.1-8% and the sulfinic acid or salt thereof is used in an amount from 0.1-2%, the combined amount ranging from about 0.2 to 10% by weight.

The amine or amine salt employed in the preferred aspects of this invention desirably is a secondary or tertiary amine rather than a primary amine, since the use of a secondary or tertiary amine leads to significantly accelerated curing. Examples of suitable amines include N,N-dimethylaniline, N,N-dimethyl-p-toluidine, N-methyl-N-$\alpha$-hydroxyethylaniline, N,N-di-$\alpha$-hydroxyethylanaline, N-methyl-analine, N-methyl-p-toluidine, dimethylamino benzoic acid and its esters and butyl diethanolamine. In a preferred aspect, the amine or amine salt comprises a (meth)acrylate group. Examples of such amines and their acrylic or methacrylic acid amine salts include dimethylaminoneopentyl (meth)acrylate, morpholinoethyl methacrylate, morpholinoethyl acrylate and the like.

The salt of sulfinic acid employed in the invention may be a salt of any organic sulfinic acid, but in terms of stability, a salt of a sulfinic acid attached to an aryl group is desirable The salts may be alkali metal salts, alkaline earth metal salts and amine salts, although the alkali metal and alkaline earth metal salts are preferred. As examples of suitable sulfinic acid salts there may mentioned sodium benzene sulfinate, calcium benzene sulfinate, triethyl ammonium benzene sulfinate, lithium toluene sulfinate, benzene sulfinic acid, N,N-dimethyl-p-toluidine sulfinate and the like. Particularly desirable are the salts of toluene sulfinic acid. The sulfinic acid or sulfinic acid salt acts as an accelerator for the catalyst system as does the amine salt and it has been found that, in a preferred aspect of this invention, both constituents should be present to ensure the desired adhesive strength and rate of polymerization of the composition.

The relative amounts of initiator, amine or amine salt and sulfinic acid or salt thereof is selected such that the composition will be shelf-stable, i.e., will not self-cure in less than about 3 months when stored at 20 °C and less than about 2 days when stored at 50 °C. In this application, a composition will be considered to have self-cured at the intended operating temperature when it has polymerized to the extent that it has become solid.

The adhesive composition optionally may include a non-reactive solvent such as an alcohol, e.g. ethanol. When used, the solvent would comprise as much as about 90% by weight of the composition.

As necessary, other polymers, fillers, stabilizers and so forth may be incorporated in the composition of this invention. As the polymers incorporated to reduce the polymerization shrinkage or for viscosity adjustment, there may be mentioned polymethyl acrylate (or methacrylate), polyethyl acrylate (methacrylate), polyhydroxyethyl methacrylate, polystyrene, unsaturated polyesters, etc. The fillers may comprise aluminum oxide, $\alpha$-quartz powders, colloidal silica, glass beads etc. within the range of about 0.04 to about 100 microns in particle diameter.

As examples of said stabilizers, there may be mentioned butylated hydroxy toluene, hydroxymethoxy benzophenone, and the like.

As will be appreciated, conventional systems which employ reactive free radical initiator systems, such as a peroxide-amine system, or a peroxide-sulfinic acid-amine system, require that the catalyst or initiator system be brought into contact with the polymerizable monomer and phosphorus acid compound only immediately before introducing the adhesive composition into a dental cavity. To this end it is common practice to put up the initiator system in two-part packs, one part containing the initiator and the other part containing the accelerator, with the polymerizable constituents being mixed with either or both of the initiator and accelerator. In accordance with the invention of the present application, however, the adhesive is formulated such that it can be put up in a single, shelf-stable pack; and in preferred embodiments the adhesive is to be polymerized by actinic radiation.

In addition to being especially suited for adhesion to dentin, the adhesive composition of the present invention adheres quite well to tooth enamel. Accordingly, the composition may be used in orthodontics, for example, to bond orthodontic brackets or bands to tooth enamel, or in periodontics, for the treatment of enamel to which a splint is to be attached.

Further, the composition may be used as an adhesive in the buildup of teeth with composite materials

and the attachment of acrylic veneers via a composite or unfilled resin for the same purpose. Similarly, they may be used as adhesives in the attachment of single unit pontics to the abutment teeth via a composite or unfilled resin.

It also has been found that the adhesive composition of the present invention may be used to adhere composite materials to metal substrates, and in this connection, the adhesive composition can find application in the construction of crowns and bridges in which a polymeric material is bonded to the framework or substrate formed of a metal, such as a chromium/cobalt alloy, a gold/copper alloy or the like, or such metals coated with reactive oxide layers as, for example, tin oxide, silicon dioxide, aluminum oxide and the like.

It is also contemplated that the primers and adhesives of this invention may be filled to such an extent that they would serve not only as primers and adhesives, but also as pit and fissure sealants and dental filling composites.

The compositions of the invention, when used as a dental sealant, comprise resin monomers, photoinitiator, reducing agent, an adhesion promoting composition and optionally inorganic fillers and a source of elutable fluoride ions and a stabilizer.

The source of elutable fluoride ions may be any suitable fluoride ion containing compound or fillers from which fluoride ion is extractable. Particularly preferred is sodium fluoride. Strontium fluoride provides a suitable source of elutable fluoride ions and may be used when it is desirable to provide a radio-opaque sealant. Examples of fluoride containing glass fillers, from which fluoride is extractable, are fluoride containing glass ionomers and barium glass.

In a dental sealant, the composition will preferably comprise by weight about 30-99% resin monomers, about 0.05-2.0% photoinitiator, about 0-2.0% reducing agent, about 0-50 vol % inorganic fillers, about 0-6% fluoride salts, and optionally about 0.05-0.2% stabilizer.

Exemplary of the resin monomers which may be used in the composition are low viscosity urethane methacrylate (U.M.), 2,2-bis-V-methacryloxy-α-hydroxyphenyl propane (Bis- GMA), triethylene glycol dimethacrylate (TEGDMA) and dipentaerythritol pentaacrylate phosphoric acid ester (PENTA - which also acts as an adhesive promoter).

PENTA and its properties are described in U.S. Patent 4,514,342 to Billington et al, said patent being incorporated herein by reference.

PENTA has been found to provide a good adhesive bond to inorganic structure of teeth and is believed to form a chemical bond with calcium ions in the tooth structure. In addition, the resin portion of the PENTA compound cross-links with the other resins in the composition on cure, to provide a base which bonds to a tooth and is structurally strong.

Those skilled in the art will recognize that an adhesive which bonds to the organic structure, such as protein, in a tooth may also be used in the composition.

As used herein, the term urethane methacrylate is generic and embraces urethane dimethacrylates.

Also, diketo photoinitiators such as camphorquinone (CQ) are preferred, although those skilled in the art will recognize that titanate and phosphonate photoinitiators may also be used.

Preferred reducing agents are organic amines such as morpholinoethyl methacrylate (MEM) and methyl diethanolamine (MDEA).

Examples of inorganic fillers that can be used in the invention are glasses known for use in dental compositions, such as glass ionomers, and other such fillers and are exemplified by barium glass, strontium oxide glass, lithium glass, silica barium sulfate, calcium phosphate, hydroxyapatite and various pigments known for use in dental compositions. Particularly desirable glasses for use in the composition are fluorine containing glasses from which fluoride ions are easily extracted.

A preferred stabilizer is butylated hydroxytoluene (BHT).

A preferred sealant composition comprises

30-70% low viscosity urethane/methacrylate monomer
0.15-0.19% Butylated hydroxytoluene
0.05-0.85% Camphorquinone
8.7-9.1% Bis GMA
0.2-0.3% Methyl Diethanolamine
0.1-0.3% Morpholinoethyl Methacrylate
6.5-7.4% Triethylene Glycol Dimethacrylate
0.8-1.2% Dipentaerythritol pentaacrylate phosphoric acid ester
20-28% Silanated Milled Barium boron aluminum silicate glass
20-28% Silanated Milled lithium aluminum silicate glass
0-1% Titanium dioxide

1.5-2.5% Sodium Fluoride and
0.1-1% fumed Silica.

In this specification the term "(meth)acry late" is intended to refer to an acrylic or methacrylic acid moiety, and the term "polymerizable monomer or prepolymer" is intended to refer to monomers, dimers, trimers or oligomers which contain ethylenic unsaturation and which may be polymerized by free radical initiation, including phosphorus derivatives containing ethylenic unsaturation. The ranges of "polymerizable monomer portion" recited herein include the phosphorus-containing adhesive promoter to the extent that the phosphorus-containing adhesive promoters are polymerizable phosphorus materials having ethylenic unsaturation. All of the percentages recited herein are by weight based on the weight of the entire composition unless otherwise stated.

Having generally described the invention, a more complete understanding can be obtained with reference to certain specific examples, which are included for purposes of illustration only. It should be understood that the invention is not limited to the specific details of the Examples.

## EXAMPLE 1

### Preparation of Urethane Dimethyacrylate (UDMA)

To a mechanically stirred mixture of 59.19 weight parts of dry hydroxypropyl methacrylate (HPMA), 0.0177 weight parts of monomethyl ether hydroquinone (MEHQ) and 0.05 weight parts of stannous octoate in a reactor, heated to 45°C and purged with dry air, there is added 40.76 weight parts of trimethylhexamethylene diisocyanate while maintaining the reaction temperature at 45° to 55°C.

After the addition is complete, the reaction mixture was held for 16 hours at 50° to 55°C and heated at 70°C until the % NCO assay is below 0.01%.

## EXAMPLE 2

### Dipentaerythritol Pentaacrylate Phosphoric Acid Ester

### (PENTA)

A solution of technical dipentaerythritol monohydroxypentaacrylate (1 mole) and triethylamine (1 mole) in dry ether was slowly added with stirring to a solution of phosphorus oxychlori (1 mole) in dry ether, at 0°C. After stirring for two hours at room temperature, the triethylamine hydrochloride formed was filtered off and the product remaining in solution was hydrolyzed by addition of the ether solution to ice water with stirring at below 10°C. The resultant mixture was separated and the separated ether layer was then extracted with a 25% aqueous sodium carbonate solution. The aqueous extract exhibited a PH of about 8. The alkaline aqueous extract was then acidified with 18% acid and an oily material was formed. The oily material was extracted with methylene chloride and the extract was dried over anhydrous sodium sulphate. The methylene chloride was then removed from the dried extract under reduced pressure to give the title compound as a clear straw-colored oil.

## EXAMPLE 3

A general procedure for the preparation of all of the resins used for the bond strength testing is illustrated as follows:

| Resin A | Weight Part |
|---|---|
| UDMA<br>Triethylene glycol dimethacrylate (TEGDMA)<br>Bisphenol A dimethacrylate (BPDMA)<br>Butylated hydroxytoluene (BHT) | 59.72<br>29.92<br>10.34<br>0.02 |

A mixture of these four components was mixed with agitation at 25 to 50C until a homogeneous solution was obtained.

| Composition B1 | Weight Part |
|---|---|
| Resin A<br>Dipentaerythritol pentaacrylate phosphoric acid ester of Example 2 (PENTA)<br>butyl diethanol amine (BDE)<br>lithium a-toluene sulfinate (LTS)<br>Camphoroquinone (CQ) | 86.59<br>9.55<br>3.38<br>0.24<br>0.24 |

This activated resin is prepared by the method described for Resin A, except that the entire operation was carried out under yellow lighting or red lighting which does not cure the resin.

## EXAMPLE 4

The following dentin bonding compositions were prepared by the method described in Example 3, except that the amine was varied as given in Table I, and evaluated in five replications by the method described below. All compositions shown are in parts by weight. The average bond strengths are illustrated in Table I.

Table I

| Composition | Resin A | PENTA | Amines | LTS | CQ | Bond (MPa) Strength | S.D. (MPa) |
|---|---|---|---|---|---|---|---|
| B1 | 86.62 | 9.55 | BDE 3.38 | 0.24 | 0.24 | 4.94 | 1.77 |
| C1 | 86.38 | 9.69 | DMANPA 3.49 | 0.22 | 0.22 | 8.08 | 3.10 |
| D1 | 86.16 | 9.18 | MPEMA 4.18 | 0.24 | 0.24 | 6.75 | 2.29 |
| E1 | 86.42 | 9.53 | MPEA 3.87 | 0.24 | 0.24 | 5.77 | 2.57 |

As shown in Table I, the amines containing a polymerizable substituent such as acrylate or methacrylate appear to afford more favorable bond strengths.

The following abbreviations in Example 4 have not been identified previously:

1. MPa is Mega Pascals.
2. DMANPA is dimethylaminoneopentyl acrylate.
3. MPEMA is 2-N-morpholinoethyl methacrylate.
4. MPEA is 2-N-morpholinoethyl acrylate.
5. S. D. is standard deviations.

## EVALUATION METHOD

Preparation of Teeth

Extracted human teeth used for the bond strength testing were treated in 1% sodium hypochlorite for 18 to 24 hours, washed with water, mechanically sanded with 120/320/600 grit carborundum paper until the dentin was exposed, and then hand sanded with 600 grit carborundum powder on glass slab. The prepared teeth were stored in distilled water in a refrigerator at about 4°C until needed.

Method of Bond Strength Test

The teeth were then individually prepared as follows: Each tooth was blow dried with compressed dry air to ensure the dentin surface was free from noticeable moisture, and the test compositions were applied with a brush and blown with compressed dry air to give a thin film on the surface. A small plastic straw with 1.84 mm of inner-diameter and 2 to 3 mm of length was filled with uncured Prisma-Fil® composite filling material (a light curing composite filling material sold by L.D. Caulk Division of Dentsply International, Inc.) and seated on the dentin so as to form a post. The upper open end of the straw was covered with a thin film of cellophane. Gentle pressure was applied to the post through the cellophane with the tip of the handpiece of a Prisma-Lite® light curing unit (sold by L.D. Caulk Division of Dentsply International, Inc.). The unit was activated and the composite was cured for 30 seconds. The specimens were stored in distilled water at 37°C for 1 to 3 days and their posts were sheared on an INSTRON with 50 kg load and 1mm/min. head speed. The shear bond strengths were calculated and are shown in the following tables.

EXAMPLE 5

The following compositions illustrate the concentration effects on bond strengths of amines shown in Table I. These resins were prepared by the method described in Example 3 and were evaluated by the method described in Example 4. The compositions shown are in weight parts and the bond strengths are summarized in the following tables. All compositions designated by the letter B, with the exception of B8, contain BDE; all compositions designated by the letter C, with the exception of C6, contain DMANPA; and all compositions designated by the letter D, with the exception of D6, contain MPEMA.

Table II

| Composition | Resin A | PENTA | BDE | LTS | CQ | Bond (MPa) Strength | S.D. (MPA) |
|---|---|---|---|---|---|---|---|
| B1 | 86.62 | 9.55 | 3.38 | 0.24 | 0.24 | 4.94 | 1.77 |
| B2 | 86.54 | 9.75 | 3.47 | 0.00 | 0.24 | 4.28 | 1.97 |
| B3 | 86.69 | 9.51 | 3.44 | 0.24 | 0.12 | 5.97 | 3.72 |
| B4 | 91.25 | 4.81 | 3.46 | 0.24 | 0.24 | 4.42 | 3.30 |
| B5 | 96.14 | 0.00 | 3.38 | 0.24 | 0.24 | 1.48 | 2.69 |
| B6 | 85.78 | 9.85 | 6.97 | 0.25 | 0.25 | 2.91 | 1.11 |
| B7 | 88.28 | 9.55 | 1.69 | 0.24 | 0.24 | 2.10 | 0.61 |
| B8 | 89.97 | 9.55 | 0.00 | 0.24 | 0.24 | 3.99 | 2.55 |

Table III

| Composition | Resin A | PENTA | DMANPA | LTS | CQ | Bond (MPA) Strength | S.D. (MPA) |
|---|---|---|---|---|---|---|---|
| C1 | 86.38 | 9.65 | 3.49 | 0.22 | 0.22 | 8.08 | 3.10 |
| C2 | 88.12 | 9.64 | 1.87 | 0.12 | 0.24 | 6.24 | 2.2 |
| C3 | 95.82 | 0.00 | 3.71 | 0.24 | 0.24 | 0.16 | 0.27 |
| C4 | 76.21 | 19.34 | 3.96 | 0.24 | 0.24 | 3.15 | 1.45 |
| C5 | 85.67 | 10.42 | 3.67 | 0.00 | 0.24 | 4.26 | 3.61 |
| C6 | 89.62 | 9.96 | 0.00 | 0.25 | 0.17 | 4.77 | 1.05 |
| C7 | 92.89 | 4.82 | 1.93 | 0.12 | 0.24 | 6.35 | 2.29 |
| *C8 | TEGDMA 46.05 | 46.48 | 6.94 | 0.17 | 0.17 | 4.89 | 1.30 |

*Use TEGDMA in place of Resin A in the composition C8 formulation.

Table IV

| Composition | Resin A | PENTA | MPEMA | LTS | CQ | Bond (MPA) Strength | S.D. (MPA) |
|---|---|---|---|---|---|---|---|
| D1 | 86.16 | 9.18 | 4.18 | 0.24 | 0.24 | 6.75 | 2.29 |
| D2 | 76.43 | 18.99 | 4.11 | 0.23 | 0.23 | 5.68 | 2.12 |
| D3 | 87.93 | 9.53 | 2.07 | 0.24 | 0.24 | 4.83 | 1.72 |
| D4 | 86.11 | 9.4 | 4.14 | 0.12 | 0.24 | 6.30 | 1.11 |
| D5 | 81.76 | 9.46 | 8.32 | 0.22 | 0.24 | 7.17 | 2.62 |
| D6 | 89.62 | 9.96 | 0.00 | 0.25 | 0.17 | 4.77 | 1.05 |
| D7 | 92.83 | 4.73 | 2.08 | 0.12 | 0.24 | 5.88 | 2.41 |

As illustrated in Tables II - IV, the following conclusions may be drawn:

1. Without PENTA in the compositions, the adhesion is very poor.
2. Sufficient adhesion can be obtained using either an amine or LTS accelerator.
3. Superior adhesion is obtained when both an amine and LTS accelerator is used.

## EXAMPLE 6

Other adhesion promoters such as polymerizable and non-polymerizable phosphates, phosphonates and phosphites in place of PENTA are illustrated in this Example. The composition preparation and test method were the same as described in Examples 3 and 4, except as otherwise indicated.

The bond strengths are summarized in Table V.

Table V

| Composition | Resin A | Adhesion Promoter | Amines | LTS | CQ | Bond (MPA) Strength | S.D. (MPA) |
|---|---|---|---|---|---|---|---|
| F | 83.93 | PETMAP 9.64 | MPEMA 5.96 | 0.24 | 0.24 | 4.02 | 1.54 |
| G | 88.1 | GPDMA 9.7 | DMANPA 1.85 | 0.24 | 0.12 | 2.68 | 3.18 |
| H | 85.95 | HEMAP 9.52 | DMANPA 4.12 | 0.23 | 0.18 | 1.66 | 1.8 |
| *I | 94.11 | BISGMAPP 4.77 | DMANPA 0.94 | 0.12 | 0.06 | 2.12 | 1.08 |
| J | 87.28 | GP 8.65 | DMANPA 3.72 | 0.23 | 0.12 | 0.5 | 1.45 |
| K | 85.92 | DP 9.4 | MPEMA 4.2 | 0.24 | 0.24 | 1.78 | 2.5 |
| L | 86 | DEHPA 9.38 | MPEMA 4.15 | 0.24 | 0.24 | 1.99 | 2.23 |
| M | 85.9 | DEHPI 9.45 | MPEMA 4.18 | 0.24 | 0.24 | 3.30 | 2.04 |
| **N | 43.07 | BISGMAP 9.66 | DMANPA 1.73 | 0.11 | 0.11 | 2.77 | 0.77 |

* Composition I was required to be mixed with an equal volume of ethanol to form a homogeneous solution and then was applied on the dentin surface.
** Composition N was mixed with an equal part of methanol and then was applied on dentin surface.

The following abbreviations in Example 6 have not been identified previously:

1. PETMAP is Petaerythritol trimethacrylate phosphoric acid ester.
2. GPDMA is glycerol 2-phosphate dimethacrylate.
3. HEMAP is 2-methacryloxyethyl phenyl phosphate.
4. BISGMAPP is BIS-GMA di (phenyl phosphonate).
5. GP is glycerol 2-phosphate.
6. DP is dibutyl phosphite.
7. DEHPA is di(2-ethylhexyl) phosphate.
8. DEHPI is di (2-ethylhexyl) phosphite.
9. BISGMAP is BIS-GMA diphosphate.

In comparing the results shown in Table V with those illustrated in Tables II, III and IV, it is apparent that the use of the polymerizable phosphates PENTA and PETMAP resulted in compositions which exhibit superior adhesion.

## EXAMPLE 8

The following compositions O, P, and Q were prepared and the stability of each composition was tested.

| Compositon O - | |
|---|---|
| Dibenzoyl Peroxide | 1 weight part |
| Polymethyl Methacrylate | 20 weight part |
| LTS | 3 weight part |

The above components were charged into a vessel and blended until homogenous. The composition O is a powder blend.

**Composition P -**

| | |
|---|---|
| CQ | 1 weight part |
| Polymethyl Methacrylate | 20 weight part |
| LTS | 3 weight part |

Composition P was prepared in the same manner as for composition O. This composition also is a powder blend.

| Composition Q - | |
|---|---|
| PENTA | 20 weight part |
| Ethylene Glycol Dimethacrylate | 20 weight part |
| N,N-Demethyl para-Toluidine | 2 weight part |

Composition Q was prepared in accordance with Example 3. This liquid composition was mixed with an equal weight part of composition O or P. The mixture of Q and O formed a solid mass instantaneously. The mixture of compositions Q and P, which were mixed and stored in the dark at room temperature, resulted in a soft unusable gel within two hours.

Composition Q, by itself, was stable when stored in a clear amber bottle at room temperature for more than two weeks. Example 8 illustrates that the simple substitution of a light cure initiator (CQ) for an acyl peroxide inititator (BPO) does not, in and of itself, result in an acceptably stable one component composition.

It is evident from the foregoing example that the invention may be described as comprising a liquid polymerizable dental adhesive composition comprising a polymerizable monomer or prepolymer, a catalyst active on command to catalyze the polymerization of the polymerizable monomer or prepolymer, and an accelerator and adhesion promoting system comprising at least two components which are balanced either in concentration or activity or both such as to provide at least three months of usable shelf life. Another characteristic of the accelerator adhesion promoting system components is that when the components are not in close tolerance balance they bring about premature polymerization of the polymerizable monomer or prepolymer or fail to bring about the polymerization of the polymerizable monomer or prepolymer within less than three minutes.

## EXAMPLE 9

## STABILITY TEST

Seven resin compositions shown in Example 5 were selected for stability study. They were kept at room temperature at 20° C to 25° C and in an oven at 50° C separately and visually inspected for whether they would gel on standing over a period of time. The results are illustrated in Table VI. Further, the shear bond strengths were determined after a week at 50C for these compositions by the method described in Example 4. The test results are summarized in Table VII.

Table VI

| Compositions | Time Elapsed | Conditioning Temperature | Physical Appearance |
|---|---|---|---|
| B1 | 2 1/2 - Month | 20° - 25° C | Liquid |
| B1 | 2 - Week | 50° C | Liquid |
| B2 | 2 1/2 - Month | 20° - 25° C | Liquid |
| B8 | 2 1/2 - Month | 20° - 25° C | Liquid |
| C1 | 2 1/2 - Month | 20° - 25° C | Liquid |
| C1 | 2 - Week | 50° C | Liquid |
| C5 | 1 1/2 - Month | 20° - 25° C | Liquid |
| C6 | 1 1/2 - Month | 20° - 25° C | Liquid |
| D1 | 2 1/2 - Month | 20° - 25° C | Liquid |
| D1 | 2 - Week | 50° C | Liquid |

Table VII

| Compositions | Bond Strength (MPa) | S.D. (MPa) |
|---|---|---|
| B1 | 4.84 | 1.82 |
| B2 | 2.21 | 1.43 |
| C1 | 8.39 | 3.77 |
| C5 | 6.19 | 3.92 |
| D1 | 7.88 | 2.50 |
| B8 | 2.56 | 1.01 |
| C6 | 1.77 | 1.16 |

EXAMPLE 10

Specific sealant compositions are illustrated in the following examples.

| COMPOSITION OF FLUORIDE SEALANT | | |
|---|---|---|
| | Tooth-colored | White |
| Raw Materials | % Wt. | % Wt. |
| Low viscosity urethane dimethacrylate Monomer | 32.653 | 32.508 |
| Butylated Hydroxy Toluene | 0.017 | 0.017 |
| Camphorquinone | 0.075 | 0.075 |
| Nupol[1] | 8.905 | 8.866 |
| Methyl Diethanolamine | 0.243 | 0.242 |
| Morpholinoethyl Methacryalte (MEM) | 0.217 | 0.216 |
| Triethylene glycol dimethacrylate | 6.927 | 6.895 |
| PENTA[2] | 0.990 | 0.985 |
| Silanated Milled Corning Glass[3] | 23.747 | 23.641 |
| Silanated Milled Cervit[4] | 23.747 | 23.641 |
| Titanium Dioxide P-25 | -- | 0.688 |
| Sodium Fluoride | 1.979 | 1.975 |
| Tullanox 300[5] | 0.50 | 0.251 |

1. NUPOL - Bis-GMA
2. PENTA - Dipentacrythridol pentaacrylate phosphoric acid ester
3. Silanated Milled Corning Glass - Barium boron aluminum silicate glass, mean particle size about 2.8 um.
4. Silanated Milled Cervit - Lithinm aluminum silicate glass
5. Tullanox 300 - fumed silica (frothy white powder).

Preparation of Low Viscosity Urethane Dimethacrylate.

To a mechanically stirred mixture 137 weight parts of 1:1 BISGMA and TEGDMA, 0.009 weight part of stannous octoate and 0.005 butylated hydroxytoluene in a reactor, heated to 45° to 50° C and purged with dry air. There is then added 33.6 weight parts of 1:1 Hexamethylene diisocyanate and TEGDMA at 50° C to 55° C.

After addition is complete, the reaction mixture was held for 16 hours at 50°-55° C until the percentage of NCO tested was below 0.01%.

EXAMPLE 11

The following is an illustrative composition of the invention which employs strontium fluoride as the source of elutable fluoride ions.

| Raw Material | % weight |
|---|---|
| Low viscosity urethane dimethacrylate monomer | 37.37 |
| Butylated Hydroxytoluene | 0.02 |
| CQ | 0.09 |
| BISGMA | 10.19 |
| Methyl Diethanolamine | 0.28 |
| MEM | 0.25 |
| TEGDMA | 7.93 |
| PENTA | 1.13 |
| Silanated Milled Corning Glass | 7.65 |
| Silanated Milled Cervit | 7.65 |
| Strontium Fluoride | 27.19 |
| Tullonox 300 | 0.25 |

EXAMPLE 12

The following illustrates an unfilled sealant of the invention.

| Raw Material | % by weight |
|---|---|
| Low viscosity urethane dimethacrylate monomer | 63.26 |
| Butylated Hydroxy Toluene | 0.03 |
| CQ | 0.15 |
| BISGMA | 17.80 |
| Methyl Diethanolamine | 0.49 |
| MEM | 0.44 |
| Triethylene glycol dimethacrylate | 13.85 |
| PENTA | 1.98 |
| Sodium Fluoride | 2.0 |

Note that the sodium flouride in this composition may be replaced with an organic amine salt of a hydroflouride to provide a more homogeneous composition.

The following table VIII illustrates some physical properties of the sealant of the invention of EXAMPLE 10 as compared to the commercial product PRISMA SHIELD, available from The L. D. Caulk Company, a Division of Dentsply International Inc. It is believed that the higher viscosity of the sealant of the invention makes it possible to apply a thicker coat of seal ant in one application because there is less sagging when the resin of the invention is applied to a vertical surface. It is noted that the depth of cure (DOC), Barcol hardness, and abrasive loss properties of the sealant of the invention are significantly better than those of the commercial product.

TABLE VIII

| PHYSICAL PROPERTY COMPARISON | | | |
|---|---|---|---|
| | | Sealant of the Invention | |
| | Prisma Shield | Tinted | Translucent |
| Viscosity[1] | 17,500 cps | 25,000 cps ± 1,500 | 23,000 cps ± 2,500 |
| Tag Length[2] (microns) | 38 ± 10 | 32 ± 13 | 38 ± 13 |
| Depth of cure[3] (20 sec) | 2 mm | 3 mm | 8 mm |
| Barcol Hardness[4] | 87 | 97 | 97 |
| Abrasive loss[5] (x $10^{-4}$) (prophy) | 41 ml | 31 ml | 33 ml |
| Sorption[6] (7 day) | 1.3 mg/cm2 | 1.5 mg/cm2 | 1.6 mg/cm2 |

1. Viscosity was measured using a Brookfield Synchro-Letric Viscometer Model LVT at 25° C. The viscometer is made by Brookfield Engineering Inc., Stoughton, MA 02072.

2. The tag length was measured as described in "Fissure Sealant: laboratories studies, Caries Reg., 8,2,(1974) by L. M. Silverstone". The tag length is length of resin penetrated into etched enamel after the sealant is cured.

3. The depth of cure was measured using the following procedure:

a. Place solid teflon plate under a plate with an 8 mm teflon hole to form a mold.

b. Using a mix stick, pack the 8 mm diameter hole of the teflon mold with composite material. (Pour sealants into the hole.)

c. Place a MYLAR™ sheet over the top of the hole.

d. Place a glass plate over the Mylar sheet and apply pressure causing flash material to ooze from the mold. Remove the glass plate.

e. Start a stopwatch and immediately irradiate the top surface of the material in the mold with PRISMETIC® Light, a product of L.D. Caulk Inc., keeping the end of light guide 2 mm away from the specimen.

f. Irradiate composites and sealants for 20 seconds.

g. Remove the Mylar sheet and demold the specimen.

h. Remove soft material from the underside of the cured specimen with a spatula or knife.

i. Square the underside of specimen by sanding with 600 paper. Measure medium barcol hardness on the underside. If hardness is not at least 70, continue sanding to remove minute increments of material from the underside, measuring hardness and repeating the process until a hardness of 70 is achieved.

* MYLAR - DuPont's Tradename of polyester film.

4. Barcol Hardness was measured using the following procedure:

a) Prepare a 20 mm of diameter 1 mm thick chip by filling sealant material in a steel ring mold between two MYLAR Sheets. Cure the sealant material with visible light from a photo flood lamp.

b) Measure the hardness of surface with a Barber-Colman Impressor, model GYZJ935 made by Barber-Colman Company, 1300 Rock Street, Rockford, IL 61101.

5. The abrasive loss was measured using the following procedure:

The abrasion resistance of a specimen of the sealant was obtained by running a standard prophylaxis on the sealant using medium prophy paste as the standard abrading substance using the following instructions:

Weigh the base (sealant), which was prepared as described in 4(a), to be used to the fourth place and record.

Set up the abrasion machine by placing either the specimen under the restraining clamp. Screw down tightly. Place a webbed prophy cup in the chuck and secure tightly. Fill the cup with the appropriate prophy paste (NUPRO (medium grit), a product of Johnson & Johnson Inc.) using a small spatula. Counter balance the machine using a 500 gm weight. Run according to chosen time schedule, alternating 30 seconds on, 15 seconds off. Check the prophy cup during each 15 second stop to make sure it has enough paste. Upon completion of the run wash the base specimen with cold water and blot dry on a paper towel. Reweigh to the fourth place. Subtract this weight from the previous weight and record the difference. Average three duplicate runs and record the average as the weight loss. Determine the specific gravity

of the specimen by ANSI/ASTM D 792-66. Divide the mean weight loss by the specific gravity to determine volume loss.

6. Sorption was measured according to ADA specification #27.

The following table IX compares the properties of commercially available PRISMA Shield with the sealant composition of the invention after extended exposures. the results of the tests illustrate superior compressive strength and improved bond to etched enamel and improved tensile strength for the sealant of the invention.

The Bond strength to etched enamel was determined as described above in Example 4. The compressive strength was determined as described by ADA specification No. 30. The tensile strength was determined as described in ADA specification #27.

TABLE IX

| PHYSICAL PROPERTY COMPARISON AFTER EXTENDED EXPOSURES | | | |
|---|---|---|---|
| | | Sealant of the Invention | |
| | Prisma Shield | Tinted | Translucent |
| Compressive strength | | | |
| Control | 28,800 psi | 36,700 | 38,700 |
| 24 hrs. boil | 29,800 | 29,700 | 39,300 |
| Thermocycled (540x, 10-50 °C) | 28,300 | 27,900 | 40,500 |
| 6 week in 37 °C $H_2O$ | 27,213 | 30,500 | 37,682 |
| Tensile strength | | | |
| Control | 4,800 psi | 4,800 | 5,200 |
| 24 hrs. boil | 5,800 | 6,000 | 7,500 |
| Thermocycled | 6,700 | 6,400 | 8,200 |
| 6 week in 37 °C $H_2O$ | 6,188 | 6,220 | 7,381 |
| Bond to etched enamel | | | |
| Control | 2,756 psi | 3,700 | 4,800 |
| 24 hrs. boil | 4,300 | 4,100 | 3,400 |
| Thermocycled | 3,400 | 3,300 | 4,600 |
| 6 week in 37 °C $H_2O$ | 2,249 | 2,070 | 2,786 |

*All test failures are in the tooth enamel - not at the sealant interface.

The following table illustrates the fluoride release properties of the base composition of the invention. The fluoride release properties are an indication of how well the base composition will facilitate the prevention of dental caries when used.

The fluoride release properties were measured as follows:

## METHOD OF FLUORIDE RELEASE MEASUREMENT

A chip of 20 mm diameter and 1 mm thick was prepared using the resin of the invention, curing the resin under photoflood lamp without a top cover for two minutes. As is common in the photo curing of resin material, oxygen inhibition prevents a full cure of the surface of the resin, and the surface of the resin remains tacky. After curing, the tacky air inhibited layer was wiped off with Kimwipe. A tiny hole was drilled in the chip. The specimen was tied with a nylon thread, suspended in 10 ml deionized water in a plastic jar, capped with a lid, and stored in 37 °C oven for 1 week, or otherwise indicated. Water in each jar was decanted to a separate 30 ml plastic beaker. The specimen in the jar was washed with 1 ml deionized

water, and the water rinse was added to the respective beaker. To the jar, 10 ml of fresh deionized water was added and put back in the 37° C oven for the next measurement. The solution was diluted with 11 ml low level TISAB * solution and measured with fluoride electrodes. The fluoride concentrations are reported in the attached tables.

*TISAB - Total Ionoic Strength Adjustor Buffer available from Orion Research Incorporated, 840 Memorial Drive, Cambridge, MA.

FLUORIDE RELEASE FOR F-SEALANT [PPM]

| Week No. | Translucent Weekly 52786 | Translucent Cumul. 52786 | Tinted Weekly 32786 | Tinted Cumul. 32786 |
|---|---|---|---|---|
| 1 | 10.4595 | 10.4595 | 6.6085 | 6.6085 |
| 2 | 0.5776 | 11.0371 | 0.7729 | 7.3814 |
| 3 | 0.2516 | 11.2887 | 0.2965 | 7.6779 |
| 4 | 0.2322 | 11.5209 | 0.2468 | 7.9247 |
| 5 | 0.7129 | 12.2338 | 0.1896 | 8.1143 |
| 6 | 0.0406 | 12.2744 | 0.1408 | 8.2551 |
| 7 | 0.0220 | 12.2964 | 0.1253 | 8.3804 |
| 8 | 0.0220 | 12.3184 | 0.1119 | 8.4923 |
| 9 | 0.0220 | 12.3404 | 0.1071 | 8.5994 |
| 10 | 0.0292 | 12.3696 | 0.0825 | 8.6819 |
| 11 | 0.0292 | 12.3988 | 0.0844 | 8.7663 |
| 12 | 0.0290 | 12.4278 | 0.1132 | 8.8795 |
| 13 | 0.0290 | 12.4568 | 0.1070 | 8.9865 |
| 14 | 0.0290 | 12.4858 | 0.6959 | 9.6824 |
| 15 | 0.0290 | 12.5148 | 0.0647 | 9.7471 |
| 16 | 0.0172 | 12.532 | 0.0130 | 9.7601 |
| 17 | 0.0172 | 12.5492 | 0.0130 | 9.7731 |
| 18 | 0.0172 | 12.5664 | 0.0130 | 9.7861 |
| 19 | 0.0172 | 12.5836 | 0.0245 | 9.8106 |
| 20 | 0.0155 | 12.5991 | 0.0245 | 9.8351 |
| 21 | 0.0155 | 12.6146 | 0.0585 | 9.8936 |
| 22 | 0.0155 | 12.6301 | 0.0598 | 9.9521 |
| 23 | 0.0155 | 12.6456 | 0.0585 | 10.0106 |

| | | | | |
|---|---|---|---|---|
| 24 | 0.0318 | 12.6774 | 0.0585 | 10.0691 |
| 25 | 0.0318 | 12.7092 | 0.0429 | 10.1120 |
| 26 | 0.0318 | 12.741 | 0.0429 | 10.1549 |
| 27 | 0.0318 | 12.7728 | 0.0429 | 10.1978 |
| 28 | 0.0318 | 12.8046 | 0.0429 | 10.2407 |
| 29 | 0.0318 | 12.8364 | 0.0176 | 10.2583 |
| 30 | 0.0318 | 12.8682 | 0.0176 | 10.2759 |
| 31 | 0.0318 | 12.9 | 0.0176 | 10.2935 |
| 32 | 0.0318 | 12.9318 | 0.0176 | 10.3111 |
| 33 | 0.0318 | 12.9636 | Steady State | |
| 34 | 0.0318 | 12.9954 | Reached | |
| 35 | 0.0318 | 13.0272 | [No Further | |
| 36 | 0.0318 | 13.059 | Measurement] | |
| . . . | . . . | . . . | . . . | . . . |
| 50 | 0.0333 | 13.5108 | | |
| 51 | 0.0333 | 13.5441 | | |
| 52 | 0.0333 | 13.5774 | | |
| 53 | 0.0244 | 13.6018 | | |
| 54 | 0.0244 | 13.6262 | | |

Having now fully described the invention, it will be apparent to one of ordinary skill in the art that many changes and modifications can be made thereto without departing from the spirit or scope of the invention as set forth in the claims.

## Claims

1. A method of treating a tooth comprising coating said tooth cavity with dental resin sealant composition which comprises at least one adhesive promoting agent.

2. The method of Claim 1 comprising coating said tooth with a sealant composition comprising elutable fluoride.

3. The method of Claim 2 comprising coating said tooth with a resin composition comprising fluoride elutable sodium fluoride.

4. The method of Claim 2 which comprises the further step of acid etching the enamel of said tooth prior to coating said tooth with said sealant.

5. The method of Claim 2 comprising coating said prepared tooth cavity with a sealant comprising by weight about

(a) 0-99% resin monomers

(b) 0.05-2.0% photoinitiator

(c) 0.2-2.0% reducing agents
(d) 0-50 vol % inorganic fillers
(e) 1.5-2.5% sodium fluoride and
(f) 0.05-0.2% stabilizer

6. The method of Claim 2 comprising coating said prepared tooth cavity with a sealant comprising by weight about
30-35% low viscosity urethane/methacrylate monomer
0.15-0.19% Butylated hydroxytoluene
0.05-0.85% Camphorquinone
8.7-9.1% Bis GMA
0.2-0.3% Methyl Diethanolamine
0.1-0.3% Morpholinoethyl Methacrylate
6.5-7.4% Triethylene Glycol Dimethacrylate
0.8-1.2% Dipentaerythritol pentaacrylate phosphoric acid ester
20-28% Silanated Milled Barium boron aluminum silicate glass
20-28% Silanated Milled lithium aluminum silicate glass
0-1% Titanium dioxide
1.5-2.5% Sodium Fluoride and
0.1-1% fumed Silica.

7. A composition for treating a prepared tooth cavity comprising a dental resin composition which comprises at least one adhesive promoting agent and elutable fluoride.

8. The composition of Claim 7 comprising a resin composition comprising elutable sodium fluoride.

9. The composition of Claim 7 comprising a dental sealant comprising by weight about
(a) 30-90% resin monomers
(b) 0.05-2.0% photoinitiator
(c) 0.2-2.0% reducing agents
(d) 0-50 vol % inorganic fillers
(e) 1.5-2.5% sodium fluoride and
(f) 0.05-0.2% stabilizer

10. The composition of Claim 7 comprising a sealant comprising by weight about
30-35% low viscosityurethane/methacrylate monomer
0.15-0.19% Butylated hydroxytoluene
0.05-0.85% Camphorquinone
8.7-9.1% Bis GMA
0.2-0.3% Methyl Diethanolamine
0.1-0.3% Morpholinoethyl Methacrylate
6.5-7.4% Triethylene Glycol Dimethacrylate
0.8-1.2% Dipentaerythritol pentaacrylate phosphoric acid ester
20-28% Silanated Milled Barium boron aluminum silicate glass
20-28% Silanated Milled lithium aluminum silicate glass
0-1% Titanium dioxide
1.5-2.5% Sodium Fluoride and
0.1-1% fumed Silica.

11. A shelf stable one component polymerizable adhesive dental sealant composition comprising a mixture of:
(a) adhesion promoting and polymerizable monomer system selected from the group consisting of at least one free radical polymerizable monomer or prepolymer having ethylenic unsaturation and a phosphorus-containing adhesion promoter which is free from halogen atoms bonded directly to a phosphorus atom and mixtures thereof, said phosphorus-containing adhesion promoter optionally containing ethylenic unsaturation and thereby optionally forming part or all of said polymerizable monomer or prepolymer; and
(b) free radical polymerization catalyst.

12. The composition of Claim 11, wherein said polymerizable monomer or prepolymer includes a derivative of at least one unsaturated carboxylic acid selected from the group consisting of (meth)acrylic acid, and itaconic acid, and mixtures thereof, and wherein said catalyst comprises a photosensitive catalyst.

13. The composition of Claim 12, wherein said polymerizable monomer or prepolymer includes at least one reaction product of a monoester of a dihydric alcohol with a polyisocyanate.

14. The composition of Claim 12 which further comprises an accelerator for said catalyst, said

accelerator being selected from the group consisting of sulfinic acid or salt thereof, amine or amine salt, and mixtures thereof, said catalyst and said accelerator being present in said composition in relative amounts such that said composition will not self-cure in less than about 3 months when stored at 20 °C and less than about 2 days when stored at 50 °C.